# FASCICULE DE BREVET EUROPEEN

(11) **EP 2 585 001 B1**
(45) Date de publication et mention de la délivrance du brevet: **13.08.2014**
(21) Numéro de dépôt: 11735514.9
(22) Date de dépôt: 17.06.2011
(51) Int. Cl.: A61F 2/38, A61B 17/64

(54) **DISPOSITIF ORTHOPEDIQUE DESTINE A EQUIPER L'ARTICULATION D'UN GENOU**
ORTHOPÄDISCHE VORRICHTUNG FÜR DAS KNIEGELENK
ORTHOPEDIC DEVICE TO BE PROVIDED ON THE KNEE JOINT

(30) Priorité: 21.10.2010 FR 1058596; 23.06.2010 FR 1055019
(43) Date de publication de la demande: 01.05.2013
(73) Titulaire: De Cortanze, André, 83150 Bandol (FR); Lecurieux-Clerville, Roger, 13008 Marseille (FR)
(72) Inventeur: De Cortanze, André, 83150 Bandol (FR); Lecurieux-Clerville, Roger, 13008 Marseille (FR)
(74) Mandataire: Novagraaf Technologies
(86) Numéro de dépôt international: PCT/FR2011/051389
(87) Numéro de publication internationale: WO 2011/161361

(56) Documents cités:
- EP-A2- 2 490 624
- WO-A-2009/134426
- WO-A1-2009/155542
- US-A- 3 779 654
- US-A- 4 637 382
- US-A1- 2004 260 302
- US-B1- 6 540 708

## Description

La présente invention a pour objet un dispositif orthopédique destiné à équiper l'articulation d'un genou.

Le domaine technique est celui des prothèses et dispositifs articulaires, spécifiques aux genoux qui nécessitent en effet des techniques particulières du fait de la complexité de l'articulation qui doit en particulier garder sa liberté de mouvement dans le plan vertical dit médian vertical tout en assurant le support du poids du corps.

Cette articulation très sollicitée peut subir des dégradations en particulier au niveau des cartilages recouvrant le condyle fémoral et le plateau tibial suite à un accident mécanique, à l'usure et/ou dues au développement d'arthrose ; il a donc été développé depuis de nombreuses années différents types de prothèses et dispositifs pour tenter de remédier à ces dégradations en remplaçant les surfaces naturelles abîmées par des implants artificiels.

On connait ainsi différentes prothèses et implants qui ont fait l'objet de nombreux dépôts de demandes de brevets telle que la demande EP 582514 de la Société Implants Orthopédiques Toutes Applications, publiée le 9 février 1994 et décrivant une prothèse totale du genou , dite à glissement composé, comportant un plateau tibial mécanique scellé dans le tibia, dont l'extrémité a été préalablement coupée, et un élément fémoral également mécanique pourvu de deux condyles disposés symétriquement de part et d'autre du plan médian vertical : cet élément fémoral, ancré dans le condyle fémoral préalablement découpé pour le recevoir, prend appui sur le plateau tibia artificiel auquel il n'est pas lié mécaniquement mais qui comporte des surfaces concaves compatibles avec celles des condyles qui viennent s'y loger, assurant ainsi le guidage de leur mouvement relatif.

Diverses autres demandes de brevets telles que la demande FR 2901690 des sociétés EVOLUTIS et OTHESIO IMPLANTS publiées le 7 décembre 2007, ou FR 2839256 de la Société SCIENCE MEDECINE publiée le 07 novembre 2003, ou encore FR 2932079 de Messieurs BERCOVY, BRACY et KERBOUL, publiée le 11 décembre 2009, décrivent aussi de telles prothèses totales du genou qui doivent « en principe permettre une reproduction fidèle du mouvement de l'articulation du genou avec une limitation de l'usure et du fluage lié aux efforts importants et répétés appliqués » aux éléments de la prothèse, et cela grâce à des choix particuliers de formes géométriques, de qualités de matériaux, d'agencements spécifiques etc, sur lesquels portent ces brevets.

Il existe également :
- des implants pour prothèses unicomportimentales du genou tel que celle décrite dans la demande de brevet FR 2908040 de la Société ASTON MEDICAL DEVELOPPEMENTS Limited et M. GIROU, publiée le 09 mai 2008, ou la demande FR 2812541 de M. AUBANIAC publiée le 08 février 2002,
- ainsi que des prothèses d'articulations du genou dites « à charnière » tel que la demande Fr 2935894 de la Société C2F IMPLANTS publiée le 19 mars 2010.

Tous ces dispositifs nécessitent cependant une résection aussi bien donc d'au moins un condyle fémoral et d'une partie du plateau tibial, qui se réalise par une opération chirurgicale très lourde pour le patient, qui est invasive, et de plus irréversible, et qui provoque des échecs mécaniques (usure, rupture, descellements, luxation, etc.) à court et moyen terme, entraînant des ré-interventions toutes aussi lourdes.

WO 2009/15552 divulgue les caractéristiques du préambule de la revendication 1.

Le problème posé est ainsi, d'une part, de remédier à ces inconvénients en évitant de telles interventions chirurgicales lourdes, avec des résections complètes et irréversibles d'au moins un condyle du fémur et d'une partie du plateau tibial et, d'autre part, de permettre une certaine réparation naturelle des dégradations de l'articulation.

Une solution au problème posé est un dispositif orthopédique destiné à équiper l'articulation d'un genou, cette articulation comprenant des premier et deuxième leviers respectivement constitués par un fémur et un tibia, dont les extrémités respectives que sont le condyle fémoral et le plateau tibial présentent des première et deuxième surfaces d'appui respectives en regard l'une de l'autre, le fémur et le tibia étant mobiles en rotation l'un par rapport à l'autre, autour de leurs surfaces d'appui respectives et dans un plan de rotation dit plan sagittal, entre une position d'extension dans laquelle le fémur et le tibia forment entre eux un premier angle d'au plus 180°, et une position de flexion dans laquelle ils forment entre eux un deuxième angle d'au moins 20 .

Ladite articulation étant sélectivement soumise à une force d'appui exercée sur les surfaces d'appui respectives desdits condyle fémoral et plateau tibial, ce dispositif orthopédique comprend des première et deuxième pièces d'appui complémentaire et non reliées directement l'une à l'autre, et des premiers et deuxièmes moyens de fixation, les première et deuxième pièces d'appui complémentaire présentant des première et deuxième surfaces d'appui complémentaire respectives ; selon l'invention lesdites première et deuxième pièces d'appui complémentaire sont fixées, dans un état opérationnel de ce dispositif orthopédique, par les premiers et deuxièmes moyens de fixation respectifs, latéralement et extérieurement, sur un même côté du condyle fémoral et du plateau tibial, de manière à s'étendre dans un plan sensiblement parallèle audit plan sagittal de rotation, et en ce que dans l'état opérationnel du dispositif orthopédique les première et deuxième surfaces d'appui complémentaire sont en contact mutuel au moins pour la position d'extension des leviers, que sont le fémur et le tibia, ce dont il résulte une réduction de ladite force d'appui exercée, dans cette position d'extension, sur les première et deuxième surfaces d'appui respectives des extrémités des leviers, fémur et tibia.

Le contour de chacune de ces pièces d'appui complémentaire, représenté par la projection de la forme de celles-ci dans un plan parallèle à celui défini par le plan sagittal de l'articulation, sur un des côtés de laquelle elles sont destinées à être fixées, comporte au moins un côté dit actif apte à venir en contact, au moins quand l'articulation est en position d'extension, contre celui de l'autre pièce.

La première pièce d'appui complémentaire, destinée à être fixée du coté du premier levier et apte à venir en appui sur celle destinée à être fixée du coté du deuxième levier, est un roulement étanche, et la deuxième pièce d'appui complémentaire est une plaque dont la surface de contact du côté actif forme une rampe sur laquelle roule la surface de contact périphérique du roulement.

Dans un mode particulier de réalisation, les formes et fixations des deux dites pièces d'appui complémentaire sont déterminées pour que celles-ci ne viennent en contact et ne s'appuient l'une contre l'autre durant la flexion de l'articulation que temporairement en extension et début de flexion des leviers, fémur et tibia, et en générant un jeu déterminé entre les surfaces des extrémités des leviers qui se font face du côté du dispositif, à savoir le plateau tibial et le condyle fémoral du genou considéré.

Un tel nouveau dispositif orthopédique destiné à équiper l'articulation d'un genou répond ainsi au problème posé car, de par sa fixation latérale et extérieure à l'articulation, l'intervention chirurgicale nécessaire est assez « légère » ; les surfaces en vis-à-vis, et venant normalement en appui, du plateau tibial d'un côté, et des condyles fémoraux de l'autre sont préservées ; de plus, les éléments du dispositif suivant l'invention, prenant les efforts d'appui au moins en position d'extension du genou et soulageant ainsi l'articulation du côté où le dispositif est installé, suppriment la douleur.

L'invention permettant également de générer un jeu déterminé entre les surfaces du plateau tibial et du condyle fémoral, il a été constaté, d'une manière surprenante pour les rhumatologues qui considèrent pourtant que les cartilages détruits le sont irréversiblement, que ceux-ci peuvent se reconstituer à condition, d'une part, de ne pas solliciter la zone concernée comme le permet donc le présent dispositif suivant l'invention et, d'autre part, que le patient suive un traitement associé.

Ainsi, en opérant par côté, et l'un après l'autre si nécessaire, on peut permettre la reconstruction des appuis naturels du genou, et enlever alors le dispositif dont les éléments ont été fixés aussi bien du côté du tibia que du côté du fémur, mécaniquement, respectivement sur le plateau tibial et le condyle fémoral, et d'une manière permettant leur enlèvement ultérieur (que l'on peut qualifier de réversible, par opposition à l'irréversibilité des dispositifs connus à ce jour tels que décrits précédemment) et donc un fonctionnement normal et sans douleur du genou sans aucun dispositif artificiel.

Préférentiellement, le dispositif suivant l'invention est destiné à être fixé d'une manière sous-cutanée et directement contre les os, sans avoir à couper une partie même latérale de ceux-ci, comme décrit ci-après, mais il peut être aussi fixé suivant deux autres modes opératoires tels que :
- l'un également sous-cutané mais avec une préparation osseuse pour réaliser, sur le côté à la fois du plateau tibial et du condyle fémoral concernés, des plans de coupe parallèles au plan médian vertical et permettant la pose des pièces du dispositif avec un contact important sur les surfaces ainsi préparées pour obtenir suivant le cas une meilleure fixation et répartition des efforts,
- l'autre mode opératoire est de positionner les deux pièces, destinées à venir en contact l'une contre l'autre, à l'extérieur de la peau et en fixant ces pièces aux os par des fixations transcutanées.

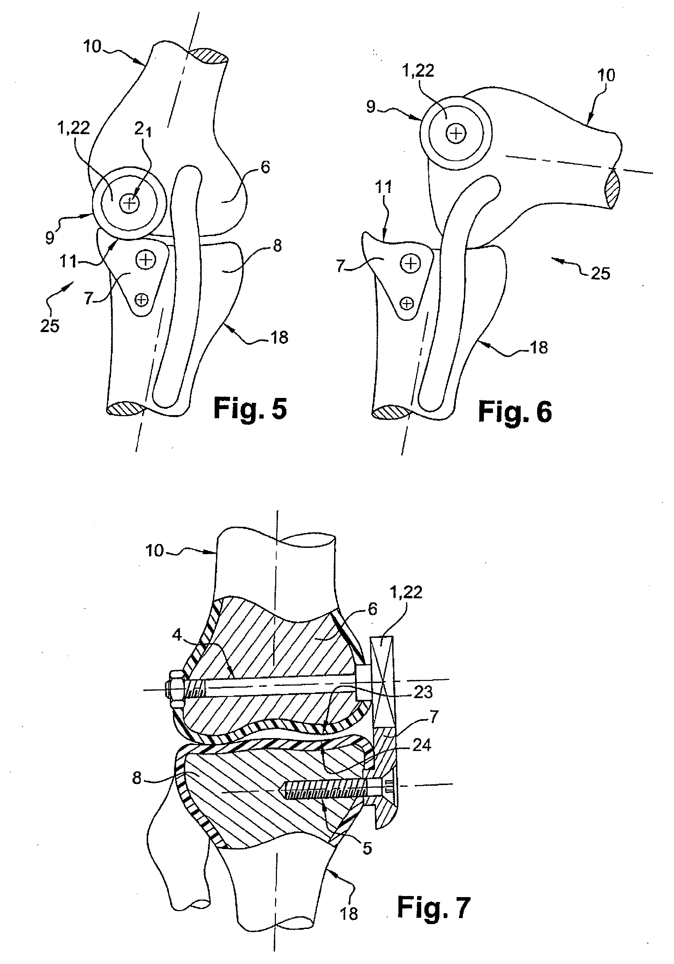

Le résultat est un nouveau dispositif orthopédique destiné à équiper l'articulation d'un genou dont les avantages évoqués ci-dessus en montrent l'intérêt et dont la description et les figures 5 à 8 ci-jointes en donnent un exemple de réalisation.

D'autres modes de réalisation sont cependant possibles dans le cadre de la portée de la présente invention.
La figure 1 représente un mode de conception théorique de base d'un dispositif ayant certaines caractéristiques de l'invention et fixé sur une articulation d'un genou en vue de côté, face au plan médian vertical en extension de celui-ci.
La figure 2 représente le même dispositif vu toujours de côté, face au plan médian vertical mais en flexion.
La figure 3 représente une coupe du dispositif suivant le plan III, III' de la figure 1, qui est perpendiculaire au plan médian vertical de l'articulation du genou.
La figure 4 représente les deux pièces principales du dispositif suivant un mode de réalisation de l'invention disposées suivant un plan' parallèle au plan médian vertical de l'articulation contre laquelle elles sont destinées à être fixées.
Les figures 5 à 7 représentent un mode de réalisation du dispositif suivant l'invention dans les mêmes configurations que celui des figures 1 à 3.
Les figures 8 représentent des vues simplifiées du dispositif selon l'invention dans la même configuration que celle de la figure 7 mais, d'une part en vue externe et non en coupe et, d'autre part, avec une caractéristique additionnelle dont le détail est représenté en grossissement sur la figure 8C.

Dans la présente description, on définit l'articulation du genou comme une articulation comprenant des premier et deuxième leviers 10, 18 respectivement constitués par un fémur 10 et un tibia 18, dont les extrémités 6, 8 respectives, que sont pour un genou respectivement le condyle fémoral et la plateau tibial, présentent des première et deuxième surfaces d'appui 23, 24 en regard l'une de l'autre.

Ces leviers 10, 18 sont mobiles en rotation l'un par rapport à l'autre, autour de leurs surfaces d'appui respectives 23, 24 et dans un plan de rotation dit plan sagittal, entre une position d'extension dans laquelle ces leviers 10, 18 forment entre eux un premier angle, entre leurs axes 13 et 21 d'au plus 180°, et une position de flexion dans laquelle ces leviers 10, 18 forment entre eux un deuxième angle d'au moins 20°, ladite articulation 25 étant sélectivement soumise à une force d'appui exercée sur les surfaces d'appui 23, 24 respectives des extrémités 6, 8 desdits leviers 10, 18.

Le dispositif orthopédique destiné à équiper cette articulation 25, telle que celle d'un genou comme représenté sur les figures, comporte ainsi au moins des première et deuxième pièces 1, 7 d'appui complémentaire et non reliées directement l'une à l'autre, et des premiers et deuxièmes moyens 2 de fixation, les première et deuxième pièces d'appui complémentaire 1, 7 présentant des première et deuxième surfaces d'appui complémentaire respectives.

Ladite deuxième pièce d'appui complémentaire 7 est apte à être fixée du côté du deuxième levier, tel que le tibia 18, et ladite première pièce 1 du côté du premier levier, tel que le fémur 10.

Suivant l'invention, ces deux dites première et deuxième pièces d'appui complémentaire 1, 7 sont fixées, dans un état opérationnel de ce dispositif orthopédique, par les premiers et deuxièmes moyens 2 de fixation respectives, latéralement et extérieurement à l'articulation du genou, sur un même côté, intérieur ou extérieur, des extrémités 6, 8 respectives, tels que le condyle fémoral et le plateau tibial, des premier 10 et deuxième 18 leviers, que sont le fémur et le tibia respectivement, de manière à s'étendre dans un plan sensiblement parallèle audit plan de rotation ou plan sagittal, et en ce que dans l'état opérationnel du dispositif orthopédique les première 9 et deuxième 11 surfaces d'appui complémentaire sont en contact mutuel au moins pour la position d'extension des leviers 10, 18, ce dont il résulte une réduction de la ladite force d'appui exercée, dans cette position d'extension, sur les première et deuxième surfaces d'appui 23, 24 respectives des extrémités 6, 8 desdits leviers 10, 18, que sont donc le condyle fémoral pour le fémur et le plateau tibial pour le tibia.

Sur l'exemple des figures, le dispositif est représenté à l'extérieur de l'articulation d'un genou, correspondant au côté du ligament interne 15, et préférentiellement d'une manière sous-cutanée, (seules les parties osseuses et un ligament étant représentées sur les figures).

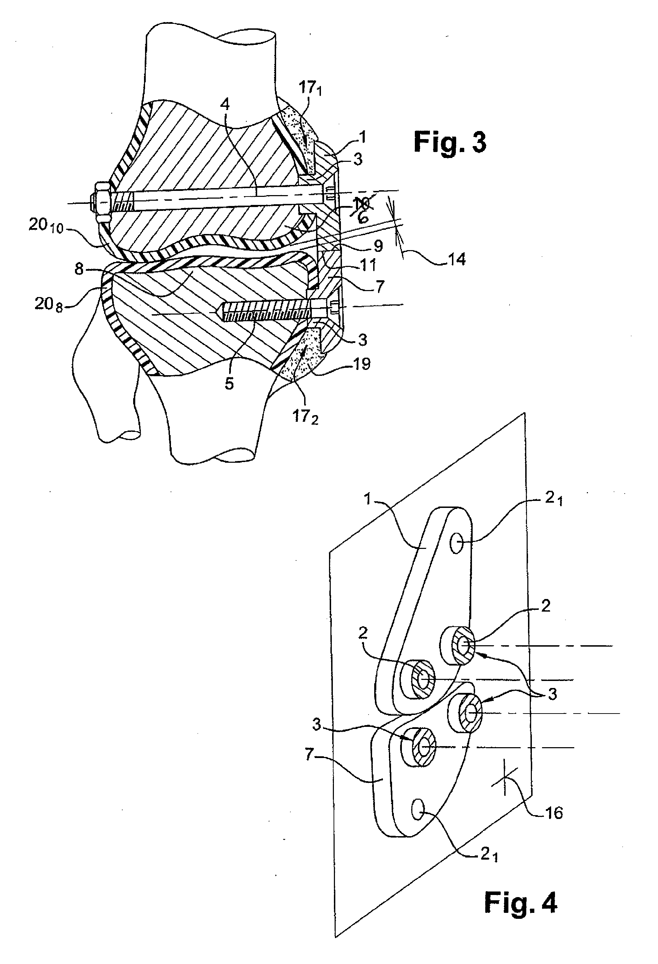

Les pièces d'appui complémentaire 1, 7 ont la forme, dans tous les modes de réalisation, de plaques dont la surface respective 9, 11 de chacune venant en contact avec celle de l'autre pièce d'appui complémentaire est réalisée dans l'épaisseur de la pièce considérée, comme représentée sur les figures 3 et 7.

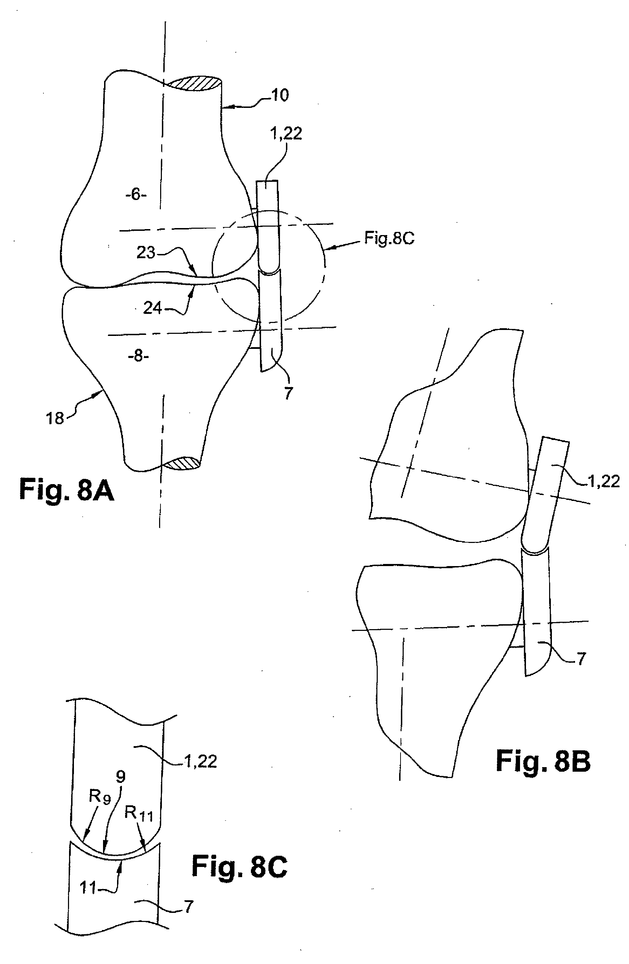

Dans un mode préférentiel de réalisation, tel que représenté sur les figures 8A, 8B et 8C, la section transversale de la surface d'appui 11 de la deuxième pièce d'appui complémentaire 7 du deuxième levier 18, tel que le tibia, est concave et la section conjuguée de la surface d'appui 9 de la première pièce d'appui complémentaire 1 du premier levier 10, tel que le fémur, est convexe avec un rayon de courbure R₉ plus petit que celui R₁₁ de la surface concave 11 de la deuxième pièce d'appui complémentaire 7 dans lequel elle coopère.

Une telle disposition permet d'obtenir un effet d'engrènement latéral stabilisateur, ce qui ne gêne pas la rotation, hors celle dans le plan sagittal, physiologique du tibia 18 par rapport au fémur 10 puisque les surfaces d'appui conjuguées ne sont déjà plus en contact quand cette rotation se produit, et par contre cette disposition empêche que lesdites pièces d'appui complémentaire 1, 7 ne puissent échapper l'une par rapport à l'autre du fait d'efforts tangentiels et de leurs surfaces de contact non forcément coplanaires, surtout lors d'efforts dissymétriques sur la jambe.

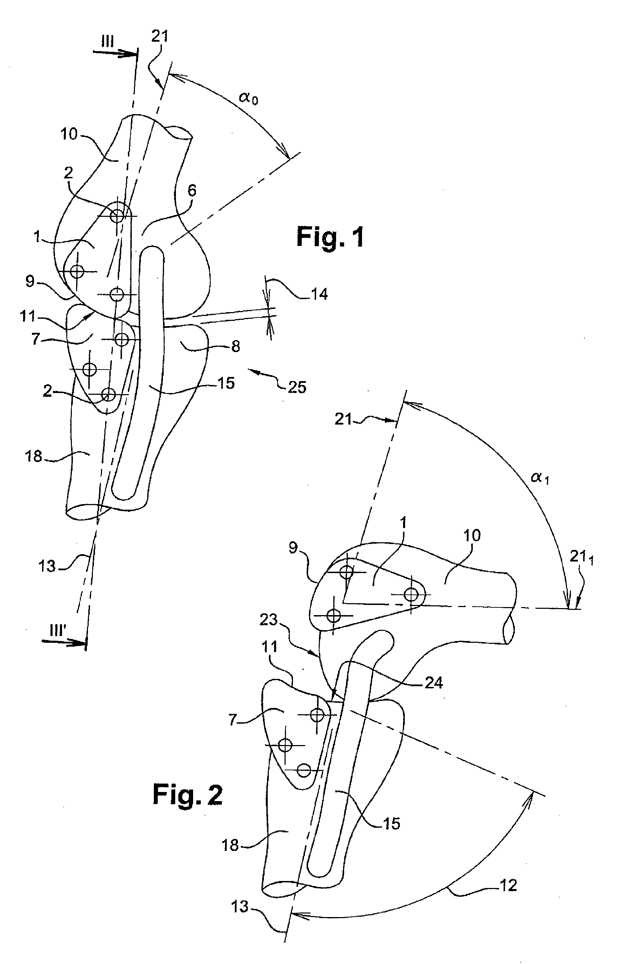

Dans le mode de conception théorique de base, tel que représenté sur les figures 1 à 3, les deux pièces d'appui complémentaire 1, 7 sont toutes les deux des plaques, rigides et monoblocs, immobilisées par rapport aux extrémités 6, 8 des leviers correspondants et dont la surface de contact du côté actif de celle 7 destinée à être fixée sur l'extrémité 8 du deuxième levier 18, tel que le plateau tibial, forme une rampe 11 sur laquelle la surface de contact du côté actif de celle 1 destinée à être fixée sur l'extrémité 6 du premier levier 10, tel que le condyle fémoral et formant une came 9, est apte à venir en appui.

Dans le mode de réalisation préférentiel de l'invention, mais qui n'est pas limitatif, d'autres modes de réalisation étant possibles, et tel que représenté sur les figures 5 à 7, la première pièce d'appui complémentaire 1 destinée à être fixée du côté du premier levier 10, soit le fémur, est apte à venir en appui sur celle destinée à être fixée du côté du deuxième levier 18, tel que le plateau tibial, est un roulement 22 étanche, défini comme étant à périphérie circulaire roulante, qu'il soit à billes, à aiguilles ou à rouleaux, et la deuxième pièce d'appui complémentaire 7 est une plaque comme dans le premier mode de réalisation précédent, dont la surface de contact du côté actif forme une rampe 11 sur laquelle roule la surface de contact 9 périphérique du roulement 22.

Un tel roulement 22 à billes, à aiguilles ou à rouleaux est circulaire et libre en rotation autour de son axe 2₁ central constituant alors son seul point de fixation 2.

Quel que soit le mode de réalisation de cette première pièce d'appui complémentaire 1 formant un roulement, il n'y a pas en ce cas, contrairement au mode de conception théorique de base, de problème de frottement de la surface d'appui 9 sur celle 11 de la deuxième pièce complémentaire 7 formant une rampe, en début de flexion de l'articulation (soit quand le point de contact de l'extrémité du premier levier 10 avec l'extrémité du deuxième levier 18, à savoir du condyle sur le plateau tibial, ne bouge pas).

Un tel roulement 22 est de préférence de deuxième génération, c'est-à-dire étanche et, de préférence, avec l'axe intégré à la bague intérieure du roulement, laquelle bague intérieure étant à double étanchéité et l'ensemble du roulement étant en matériaux biocompatibles ; la forme de ladite bague intérieure, en contact avec la peau, a une forme évitant que le frottement, au cours de la flexion, soit irritante.

La fixation desdites pièces 1, 7 d'appui complémentaire est définie pour être assurée perpendiculairement au plan médian vertical de toute articulation, tel qu'un genou, sur les extrémités 6, 8 des leviers de laquelle elles sont destinées à être fixées latéralement : les premiers et deuxièmes moyens de fixation 2 de ces pièces 1, 7 doivent permettre une fixation mécanique et d'une manière permettant leur enlèvement ultérieur.

Pour cela, ces pièces d'appui complémentaire comportent chacune un ou plusieurs alésages de fixation 2, de préférence deux ou trois quand il s'agit de plaques proprement dites de type monobloc, comme sur les figures 1 à 4, ou pour une des pièces des figures 5 à 7, et bien sûr un seul pour le roulement du mode de réalisation des figures 5 à 7 : cet alésage 2 débouche, au moins pour l'un entre eux, dans un bossage de localisation et de blocage de la pièce considérée.

Ainsi, chacune des pièces d'appui complémentaire 1, 7 comporte au moins un bossage 3 faisant saillie par rapport à la face de la pièce destinée à venir en appui latéral contre l'extrémité 6, 8 du levier considéré 10, 18, soit pour l'une le fémur et pour l'autre le tibia, et est apte à être fixée par au moins un boulon 4 traversant le bossage 3, lequel boulon 4 formant l'axe de rotation 2₁, dans le mode de réalisation correspondant, du roulement 22 ; lequel bossage est destiné à venir se bloquer dans un trou réalisé dans la paroi 20 de ladite extrémité 6, 8 des leviers 10, 18, soit la corticale 20 de l'os correspondant, et prenant en sandwich cette extrémité 6, 8, soit pour l'une le tibia et pour l'autre le fémur, comme représenté sur les figures 3 et 7.

Comme il peut ne pas être nécessaire que chaque alésage 2 comporte un bossage 3, cela pouvant dépendre du poids de la personne concernée et de la densité osseuse rencontrée, les dites pièces 1, 7 d'appui complémentaire sont aptes à être fixées, quand il s'agit de plaques proprement dites, respectivement sur un des côtés des extrémités 6, 8 des leviers 10, que sont le tibia et le fémur, par au moins une vis 5 non traversant venant se visser et se bloquer dans la paroi 20 de l'extrémité du levier correspondant, soit la corticale 20, et dans le corps de ces extrémités tel que l'os spongieux, respectivement du fémur et du tibia, comme représenté sur les figures 3 et 7.

Les bossages 3, quand les pièces 1, 7 d'appui complémentaire en comportent au moins deux et sont positionnées l'une par rapport à l'autre, prêtes à être mis en place comme représenté sur la figure 4, génèrent un plan 16 de mise en place pour le praticien, qui garantit le positionnement relatif des dites pièces.

Le contour de chacune de ces pièces 1, 7 d'appui complémentaire, qui dans tous les cas, comme indiqué précédemment, sont en forme de plaques, que ce soient des plaques proprement dites, ou des roulements, représenté par la projection de la forme de celles-ci dans un plan parallèle, qui peut être le plan 16 défini précédemment, à celui défini par le plan sagittal de l'articulation 25, qui peut être le plan médian vertical du genou, sur un des côtés de laquelle elles sont destinées à être fixées, comporte un côté dit actif apte à venir en contact, au moins quand l'articulation 25 est au moins en position d'extension, contre celui de l'autre pièce complémentaire d'appui : la surface de contact du côté actif de celle 7 destinée à être fixée sur l'extrémité 8 du deuxième levier 18, soit côté tibia, forme une rampe 11 sur laquelle la surface de contact, qui est soit en forme de came 9, soit circulaire dans le cas d'un roulement 22, du côté actif de celle 1 destinée à être fixée sur l'extrémité du premier levier 10, soit côté fémur, est apte à venir en appui.

A titre indicatif mais non limitatif, les dimensions des pièces 7, 1 peuvent être telles qu'elles s'inscrivent chacune dans un parallélépipède rectangle d'environ 40x45x15 mm, bossages 3 et moyens de fixation tels que les vis 5 et boulons 4 non compris.

Selon certaines réalisations, la came 9 ou roulement 22, et la rampe 11 peuvent être réalisées dans des matériaux différents, selon que l'on veut privilégier la résistance mécanique, le frottement ou au contraire la réduction de ce frottement, ou encore l'usure du dispositif ; les surfaces de contact 9, 11 peuvent être également garnies de matériaux antifriction, plastique, composite ou céramique, la liste n'étant pas exhaustive.

Le contour de chacune de ces deux pièces 1, 7 est tel que la came 9 ou roulement 22, de l'une 1 est apte à ne s'appuyer donc sur la rampe 11 de l'autre qu'en extension et en début de flexion de l'articulation 25 sur un des côtés de laquelle elles sont fixées. Ainsi, la came 9 ou roulement 22 s'appuie temporairement sur la rampe 11 pendant les mouvements de l'articulation 25, entre la position d'extension, pour laquelle l'axe 21 du premier levier 10, tel que le fémur, est le plus vertical possible tel que représentée sur les figures 1 et 5, jusqu'à une flexion d'un angle α₀ de 40° environ, positions entre lesquelles cet appui permet de générer un jeu 14 entre les surfaces 6, 8 d'extrémités des premier et deuxième leviers 10, 18, soit entre les surfaces du condyle fémoral 6 du plateau tibial 8, du côté où le dispositif est installé, comme représenté sur les figures 1 3, 5 et 7.

Dans le cas de la conception théorique de base d'une première pièce d'appui complémentaire qui est une plaque 1 proprement dite, de type monobloc, comme sur les figures 1 à 3, le profil de la came 9 est déterminé en fonction de l'amplitude de flexion de l'articulation 25 au cours de laquelle on souhaite supprimer le contact entre les surfaces des extrémités des premier et deuxième leviers, soit du condyle et du plateau tibial et, de ce fait, pouvoir régénérer du cartilage dans cette zone qui n'est plus soumise aux efforts : ce profil peut avoir différents rayons de courbure pour tenir compte de la variété et de l'étendue des pathologies des genoux à traiter.

La deuxième pièce d'appui complémentaire 7 peut comporter une surface de contact de la rampe 11 présentant des angles d'attaque 12 différents en fonction du point de rattrapage du contact physiologique normal condyle/plateau tibial qui a été choisi par le chirurgien ; l'angle d'attaque 12 est défini par l'angle que forme la surface de contact 11 avec l'axe 13 du deuxième levier 18, qui est le tibia, alors que l'angle α de flexion du premier levier 10, qui est le fémur, est déterminé entre l'axe 21 de celui-ci en position d'extension et ce même axe 21₁ dans la position de flexion considérée qui peut aller jusqu'à un angle α₁ de 90 à 110°.

Entre la position de flexion α₀ et cette position extrême α₁, la surface de l'extrémité, qui est le condyle fémoral 6, du premier levier reprend donc contact avec la surface de l'extrémité qui est le plateau tibial 8, du deuxième levier, ce qui permet de préserver les cartilages postérieurs qui nécessitent, en effet, de rester en contact pour cela.

Les surfaces de contact entre la came 1 ou roulement 22 et la rampe 7 ne sont restreintes en mobilité l'une par rapport à l'autre que selon un degré de liberté, ce qui autorise la rotation physiologique naturelle du tibia par rapport au fémur.

Et le montage/démontage mécanique des pièces du dispositif permet de procéder à des échanges de la paire de pièces came 1 ou roulement 22 et rampe 7 pour tenir compte de l'évolution du traitement et de la réduction contrôlée et progressive du jeu 14 entre le condyle 10 et le plateau tibial 8 au fur et à mesure que le cartilage a pu se régénérer : à la fin du traitement, l'ablation et l'enlèvement complet de ces pièces ne laisse aucune séquelle articulaire pour le patient.

Selon l'invention, et tel que représenté sur la figure 3, pour obtenir une continuité de surface entre les pièces du dispositif, hors bien sûr du côté du roulement 22, fixées mécaniquement sur le plateau tibial et le condyle fémoral, et la surface du tibia et du fémur respectivement, afin d'assurer le confort cutané, le dispositif comporte du ciment 19 venant combler des interstices résiduels 17 entre le contour des dites pièces 7, 1 et la surface latérale des os sur lesquels ces pièces sont fixées.

## Revendications

1. Dispositif orthopédique destiné à équiper l'articulation (25) d'un genou, cette articulation comprenant des premier et deuxième leviers (10, 18) respectivement constitués par un fémur (10) et un tibia (18), dont les extrémités (6, 8) respectives présentent des première et deuxième surfaces d'appui (23, 24) en regard l'une de l'autre, ces leviers (10, 18) étant mobiles en rotation l'un par rapport à l'autre, autour de leurs surfaces d'appui respectives (23, 24) et dans un plan de rotation dit plan sagittal, entre une position d'extension dans laquelle ces leviers (10, 18) forment entre eux un premier angle d'au plus 180°, et une position de flexion dans laquelle ces leviers (10, 18) forment entre eux un deuxième angle d'au moins 20°, ladite articulation (25) étant sélectivement soumise à une force d'appui exercée sur les surfaces d'appui (23, 24) respectives des extrémités (6, 8) desdits leviers (10, 18), ce dispositif orthopédique comprenant des première et deuxième pièces (1, 7) d'appui complémentaire et non reliées directement l'une à l'autre, et des premiers et deuxièmes moyens (2) de fixation, les première et deuxième pièces d'appui complémentaire (1, 7) présentant des première et deuxième surfaces d'appui complémentaire respectives, dans lequel :
- lesdites première et deuxième pièces d'appui complémentaire (1, 7) sont aptes à être fixées, dans un état opérationnel de ce dispositif orthopédique, par les premiers et deuxièmes moyens (2) de fixation respectifs, latéralement et extérieurement, sur un même côté des extrémités (6, 8) respectives des premier (10) et deuxième (18) leviers, de manière à s'étendre dans un plan sensiblement parallèle audit plan de rotation, et en ce que dans l'état opérationnel du dispositif orthopédique les première (9) et deuxième (11) surfaces d'appui complémentaire sont en contact mutuel au moins pour la position d'extension des leviers (10, 18), ce dont il résulte une réduction de ladite force d'appui exercée, dans cette position d'extension, sur les première et deuxième surfaces d'appui (23, 24) respectives des extrémités (6, 8) desdits leviers (10, 18)
- le contour de chacune de ces pièces d'appui complémentaire (1,7), représenté par la projection de la forme de celles-ci dans un plan parallèle (16) à celui défini par le plan sagittal de l'articulation (25), sur un des côtés de laquelle elles sont destinées à être fixées, comporte au moins un côté dit actif apte à venir en contact, au moins quand l'articulation (25) est en position d'extension, contre celui de l'autre pièce,
**caractérisé en ce que**
la première pièce d'appui complémentaire (1), destinée à être fixée du coté du premier levier (10) et apte à venir en appui sur celle (7) destinée à être fixée du coté du deuxième levier (18), est un roulement (22) étanche, et la deuxième pièce d'appui complémentaire (7) est une plaque dont la surface de contact du côté actif forme une rampe (11) sur laquelle roule la surface de contact (9) périphérique du roulement (22).

2. Dispositif suivant la revendications 1, **caractérisé en ce que** les formes des deux dites pièces d'appui complémentaire(1, 7) sont déterminées pour que celles-ci ne viennent en contact et ne s'appuient l'une contre l'autre, durant la flexion de l'articulation (25), que temporairement en extension et début de flexion de celle-ci et en générant un jeu (14) déterminé entre les surfaces (9, 11) des extrémités (6, 8) des leviers (10, 18) qui se font face du côté du dispositif.

3. Dispositif suivant l'une quelconque des revendications 1 à 2, **caractérisé en ce que** la section transversale de la surface d'appui (11) de la deuxième pièce d'appui complémentaire (7) du deuxième levier (18) est concave et la section conjuguée de la surface d'appui (9) de la première pièce d'appui complémentaire (1) du premier levier (10) est convexe avec un rayon de courbure (R₉) plus petit que celui (R₁₁) de la surface concave (11) de la deuxième pièce d'appui complémentaire (7) dans laquelle elle coopère.

4. Dispositif suivant l'une quelconque des revendications 1 à 3, **caractérisé en ce que** les pièces d'appui complémentaire (1, 7)) aptes à être fixées latéralement sur les extrémités (6, 8) des leviers (10, 18) le sont mécaniquement et d'une manière permettant leur enlèvement ultérieur.

5. Dispositif suivant l'une quelconque des revendications 1 à 4, **caractérisé en ce que** au moins l'une des pièces d'appui complémentaire (1, 7) comporte au moins un bossage (3) faisant saillie par rapport à la face de la pièce destinée à venir en appui latéral contre l'extrémité (6, 8) du levier considéré (10, 18), et est apte à être fixée par au moins un boulon (4) traversant le bossage (3), lequel bossage est destiné à venir se bloquer dans un trou réalisé dans la paroi (20) de la dite extrémité (6, 8) de levier (10, 18), et prenant en sandwich celle-ci.

6. Dispositif suivant l'une quelconque des revendications 1 à 5, **caractérisé en ce que** les dites pièces d'appui complémentaire (1, 8) sont aptes à être fixées respectivement sur un des côtés des extrémités (6, 8) des leviers (10, 18) par au moins une vis (5) non traversant et venant se bloquer dans la paroi (20) de l'extrémité du levier correspondant.

7. Dispositif suivant la revendication 1 à 6, **caractérisé en ce que** le roulement (22) à billes, à aiguilles ou à rouleaux est circulaire et libre en rotation autour de son axe (2₁) central constituant son seul point de fixation (2).

## Patentansprüche

1. Orthopädische Vorrichtung zum Ausstatten eines Kniegelenks (25), wobei dieses Gelenk erste und zweite Hebel (10, 18) umfasst, die jeweils aus einem Oberschenkel (10) und einem Schienbein (18) bestehen, deren jeweilige Enden (6, 8) einander gegenüberliegende erste und zweite Passflächen (23, 24) aufweisen, wobei diese Hebel (10, 18) im Verhältnis zueinander um ihre jeweiligen Passflächen (23, 24) herum und in einer Drehebene, die als Sagittalebene bezeichnet wird, zwischen einer gestreckten Position, in der diese Hebel (10, 18) untereinander einen ersten Winkel von höchstens 180° bilden, und einer gebeugten Position, in der diese Hebel (10, 18) untereinander einen zweiten Winkel von mindestens 20° bilden, drehbeweglich sind, wobei das Gelenk (25) selektiv einer Stützkraft ausgesetzt wird, die auf die jeweiligen Passflächen (23, 24) der Enden (6, 8) der Hebel (10, 18) dieser orthopädischen Vorrichtung ausgeübt wird, die erste und zweite sich ergänzende Passteile (1, 7), die nicht direkt miteinander verbunden sind, und erste und zweite Befestigungsmittel (2) umfasst, wobei die ersten und zweiten sich ergänzenden Passteile (1, 7) jeweilige erste und zweite sich ergänzende Passflächen aufweisen, wobei
- die ersten und zweiten sich ergänzenden Passteile (1, 7) dazu geeignet sind, in einem Betriebszustand dieser orthopädischen Vorrichtung durch die jeweiligen ersten und zweiten Befestigungsmittel (2) seitlich und äußerlich auf derselben Seite der jeweiligen Enden (6, 8) der ersten (10) und zweiten (18) Hebel befestigt zu sein, um sich in einer Ebene zu erstrecken, die im Wesentlichen parallel zu der Drehebene ist, und wobei im Betriebszustand der orthopädischen Vorrichtung die ersten (9) und zweiten (11) sich ergänzenden Passflächen mindestens für die gestreckte Position der Hebel (10, 18) in gegenseitigem Kontakt stehen, woraus sich in dieser gestreckten Position eine Reduzierung der ausgeübten Stützkraft auf die jeweiligen ersten und zweiten Passflächen (23, 24) der Enden (6, 8) der Hebel (10, 18) ergibt,
- der Umriss jedes dieser sich ergänzenden Passteile (1, 7), der durch die Projektion ihrer Form in einer Ebene dargestellt ist, die parallel (16) zu derjenigen ist, die durch die Sagittalebene des Gelenks (25), auf einer Seite desselben, auf der sie zu befestigen sind, definiert wird, mindestens eine so genannte aktive Seite umfasst, die dazu geeignet ist, mindestens dann, wenn sich das Gelenk (25) in der gestreckten Position befindet, mit der Seite des anderen Teils in Kontakt zu kommen,
**dadurch gekennzeichnet, dass** das erste ergänzende Passteil (1), das dazu gedacht ist, auf der Seite des ersten Hebels (10) befestigt zu sein, und geeignet ist, sich auf diesem Teil (7) abzustützen, das dazu gedacht ist, auf der Seite des zweiten Hebels (18) befestigt zu sein, ein dichtes Wälzlager (22) ist, und das zweite ergänzende Passteil (7) eine Platte ist, deren Kontaktfläche auf der aktiven Seite eine Rampe (11) bildet, auf der die umlaufende Kontaktfläche (9) des Wälzlagers (22) abrollt.

2. Vorrichtung nach Anspruch 1, **dadurch gekennzeichnet, dass** die Formen der beiden sich ergänzenden Passteile (1, 7) bestimmt werden, damit sich diese während der Beugung des Gelenks (25) nur zeitweise im gestreckten Zustand und zu Beginn seiner Beugung berühren und aufeinanderliegen und dabei ein bestimmtes Spiel (14) zwischen den Oberflächen (9, 11) der Enden (6, 8) der Hebel (10, 18) generieren, die sich auf der Seite der Vorrichtung gegenüberliegen.

3. Vorrichtung nach einem der Ansprüche 1 bis 2, **dadurch gekennzeichnet, dass** der Querschnitt der Passfläche (11) des zweiten ergänzenden Passteils (7) des zweiten Hebels (18) konkav ist und der passende Querschnitt der Passfläche (9) des ersten ergänzenden Passteils (1) des ersten Hebels (10) konvex ist mit einem Krümmungsradius (R₉), der kleiner ist als derjenige (R₁₁) der konkaven Oberfläche (11) des zweiten ergänzenden Passteils (7), in dem es zusammenwirkt.

4. Vorrichtung nach einem der Ansprüche 1 bis 3, **dadurch gekennzeichnet, dass** die ergänzenden Passteile (1, 7), die geeignet sind, seitlich an den Enden (6, 8) der Hebel (10, 18) befestigt zu sein, mechanisch und auf eine Art und Weise, die ihr späteres Abnehmen ermöglicht, befestigt sind.

5. Vorrichtung nach einem der Ansprüche 1 bis 4, **dadurch gekennzeichnet, dass** mindestens eines der sich ergänzenden Passteile (1, 7) mindestens eine Erhebung (3) umfasst, die im Verhältnis zu der Seite des Teils vorsteht, die dazu gedacht ist, seitlich am Ende (6, 8) des betreffenden Hebels (10, 18) aufzuliegen, und geeignet ist, durch mindestens einen Bolzen (4), der durch die Erhebung (3) geht, befestigt zu werden, wobei die Erhebung dazu gedacht ist, sich in einem Loch festzusetzen, das in der Wand (20) des Endes (6, 8) des Hebels (10, 18) ausgebildet ist, um dieses einzuklemmen.

6. Vorrichtung nach einem der Ansprüche 1 bis 5, **dadurch gekennzeichnet, dass** die sich ergänzenden Passteile (1, 8) in der Lage sind, jeweils auf einer der Seiten der Enden (6, 8) der Hebel (10, 18) durch mindestens eine Schraube (5) befestigt zu werden, die nicht durchgehend ist und sich in der Wand (20) des Endes des entsprechenden Hebels festsetzt.

7. Vorrichtung nach Anspruch 1 bis 6, **dadurch gekennzeichnet, dass** das Kugellager (22), das Nadellager oder das Rollenlager kreisförmig und um seine mittlere Achse (2₁) herum freidrehend ist, die seinen einzigen Befestigungspunkt (2) bildet.

## Claims

1. An orthopedic device intended to be fitted to a knee joint (25), this joint comprising first and second levers (10, 18) consisting respectively of a femur (10) and a tibia (18), of which the respective ends (6, 8) have first and second bearing surfaces (23, 24) facing one another, these levers (10, 18) being mobile in rotation relative to one another about their respective bearing surfaces (23, 24) and in a plane of rotation, known as the sagittal plane, between an extended position in which these levers (10, 18) make a first angle of at most 180° between them and a flexed position in which these levers (10, 18) make a second angle of at least 20° between them, said joint (25) being selectively subjected to a bearing force applied to the respective bearing surfaces (23, 24) of the ends (6, 8) of said levers (10, 18), this orthopedic device comprising first and second bearing pieces (1, 7) that complement one another and are not directly connected together, and first and second fixing means (2), the first and second complementary bearing pieces (1, 7) having first and second respective complementary bearing surfaces, wherein:
- said first and second complementary bearing pieces (1, 7) are able to be fixed, when this orthopedic device is in an operational state, by the first and second respective fixing means (2), laterally and externally, on one and the same side of the respective ends (6, 8) of the first (10) and second (18) levers so that they are positioned in a plane substantially parallel to said plane of rotation, and when the orthopedic device is in the operational state, the first (9) and second (11) complementary bearing surfaces are in mutual contact at least when the levers (10, 18) are in the extended position, the consequence of this being a reduction in said bearing force applied, in this extended position, to the first and second respective bearing surfaces (23, 24) of the ends (6, 8) of said levers (10, 18),
- each of these complementary bearing pieces (1, 7), has a contour represented by the projection of the shape of these pieces in a plane parallel (16) to the plane defined by the sagittal plane of the joint (25), on one of the sides of which they are intended to be fixed, which contour comprises at least one side known as the active side able to come into contact, at least when the joint (25) is in the extended position, with the contour side of the other piece,
**characterized in that** the first complementary bearing piece (1), intended to be fixed on the side of the first lever (10) and able to bear against the piece (7) intended to be fixed on the side of the second lever (18), is a sealed rolling bearing (22), and the second complementary bearing piece (7) is a plate of which the active side contact surface forms a ramp (11) over which the peripheral contact surface (9) of the rolling bearing (22) rolls.

2. The device as claimed in claim 1, **characterized in that** the shapes of the two said complementary bearing pieces (1, 7) are determined so that these come into contact with and bear against one another, when the joint (25) is being flexed, only temporarily during extension and at the start of flexing of this joint and at the same time generating a set clearance (14) between the surfaces (9, 11) of the ends (6, 8) of the levers (10, 18) which face one another on the side of the device.

3. The device as claimed in either one of claims 1 and 2, **characterized in that** the bearing surface (11) of the second complementary bearing piece (7) of the second lever (18) has a transverse section which is concave and the bearing surface (9) of the first complementary bearing piece (1) of the first lever (10) has a mating section which is convex with a radius of curvature (R₉) smaller than that (R₁₁) of the concave surface (11) of the second complementary bearing piece (7) in which it engages.

4. The device as claimed in any one of claims 1 to 3, **characterized in that** the complementary bearing pieces (1, 7) able to be fixed laterally to the ends (6, 8) of the levers (10, 18) are mechanically fixed and in a way that allows their subsequent removal.

5. The device as claimed in any one of claims 1 to 4, **characterized in that** at least one of the complementary bearing pieces (1, 7) comprises at least one embossing (3) projecting from the face of the piece that is intended to bear laterally against the end (6, 8) of the relevant lever (10, 18) and is able to be fixed by at least one bolt (4) passing through the embossing (3), which embossing is intended to be immobilized in a hole made in the wall (20) of said end (6, 8) of lever (10, 18), and sandwiching this end.

6. The device as claimed in any one of claims 1 to 5, **characterized in that** said complementary bearing pieces (1, 8) are able to be fixed respectively on one of the sides of the ends (6, 8) of the levers (10, 18) by at least one non-traversing screw (5) that becomes immobilized in the wall (20) of the end of the corresponding lever.

7. The device as claimed in claim 1 to 6, characterized the sealed rolling bearing (22), which is ball, needle or roller type, is circular and free to rotate about its central axis (2₁) that is the only point of fixation (2) thereof.
